# EUROPEAN PATENT APPLICATION

(11) **EP 3 165 249 A1**
(43) Date of publication of application: **10.05.2017**
(21) Application number: 15815540.8
(22) Date of filing: 26.05.2015
(51) Int. Cl.: A61M 5/32

(54) **SAFETY NEEDLE ASSEMBLY**

(30) Priority: 02.07.2014 JP 2014137253
(71) Applicant: Terumo Kabushiki Kaisha, Tokyo 151-0072 (JP)
(72) Inventor: KAWANO, Naoki, Nakakoma-gun Yamanashi 409-3853 (JP); KUROSE, Katsutoshi, Tokyo 163-1450 (JP)
(74) Representative: Casalonga
(86) International application number: PCT/JP2015/065040
(87) International publication number: WO 2016/002389

(57) **Abstract**

A safety needle assembly includes a hub including a proximal end connected to a syringe and a distal end, a cannula having a thickness of 18 to 24 gauge and a length between the hub and a distal end of 8 to 22 mm, and a sheath which houses the cannula. The sheath includes a projecting stopper for restricting movement, toward a distal end of the sheath, of a finger of a user pushing the sheath for housing the cannula into the opening of the sheath. The sheath includes a rib which guides the cannula introduced into the opening and a guide rib which has a locking protrusion for locking the cannula with an angle of 0° or more with respect to a housing direction of the cannula after the cannula has passed the locking protrusion.

## Description

### Technical Field

The present invention relates to a safety needle assembly that is used in subcutaneous injection of a high viscosity formulation, and more particularly, to a safety needle assembly that ensures safety in subcutaneous injection of a high viscosity formulation.

### Background Art

Conventionally, injection has been employed as a method for delivering therapeutic agents into the body of a patient. Examples of the injection include intradermal injection, subcutaneous injection, intramuscular injection, intravenous injection, arterial injection, intraarticular injection, and epidural injection. Among these injections, for example, subcutaneous injection is used in diseases such as diabetes and rheumatism. Patent Literature 1 proposes a subcutaneous infusion system as a system that performs subcutaneous in j ection. The subcutaneous infusion system is provided with a needle of 24 to 27 gauge or 18 gauge and a medication dispensing device. The needle includes a shaft having an internal duct of unvarying diameter defining a fluid pathway between openings at distal and proximal ends of the needle. A hub in fluid communication with the duct is disposed at the distal end of the needle, and the needle length from the hub to the proximal end is approximately 4 to 6 mm. The medication dispensing device is in detachable fluid communication with the needle duct and contains approximately 3 to 100 mls of a highly viscous therapeutic fluid composition. The subcutaneous infusion system of Patent Literature 1 is configured for subcutaneous delivery of the therapeutic fluid composition at a flow rate of approximately 1 to 20 ml per minute.

### Citation List

### Patent Literature

Patent Literature 1: JP 2012-509749 W

### Summary of Invention

### Technical Problem

Health care workers have to safely handle subcutaneous injection needles. For example, if a health care worker erroneously sticks a subcutaneous injection needle used for a patient or the like into the own body (finger, for example), the health care worker may be infected with a disease. However, the system of Patent Literature 1 has no means for preventing such a sting accident and thus cannot be perfectly safe. There may be a method, as safety measure, that covers a used subcutaneous injection needle with a cylindrical cap. However, even in this method, a health care worker may erroneously stick the needle into his finger holding the cap. Therefore, this method also cannot be perfectly safe.

It is an object of the present invention to solve the above problem associated with conventional techniques and provide a safety needle assembly that ensures safety in subcutaneous injection of a high viscosity formulation.

### Solution to Problem

In order to solve the above issue, the present invention provides a safety needle assembly including: a hub including a proximal end that is configured to be connected to a syringe and a distal end; a cannula including a proximal end that is configured to be connected to the distal end of the hub, a lumen extending in a longitudinal direction of the cannula, and a distal end, the cannula having a thickness of range of 18 to 24 gauge and a length between the hub and the distal end of range of 8 to 22 mm; a collar attached to the hub and including a sheath attachment part; a protector configured to cover the distal end of the cannula by being attached to the hub or the collar, the protector being detachable for exposing the distal end of the cannula; and a sheath including an opening extending along at least a part of the sheath in a longitudinal direction, the opening being capable of housing the cannula, the sheath further including: a cannula fixing part configured to fix the cannula, the cannula fixing part extending along at least a part of the sheath in the longitudinal direction; an attachment part configured to rotatably support the sheath in a direction of housing the cannula in the opening when the protector is detached to expose the distal end of the cannula, the attachment part being attached to the sheath attachment part of the collar; a projecting stopper for restricting movement, toward a distal end of the sheath, of a finger of a user pushing the sheath for housing the cannula into the opening, the stopper being disposed near a central part of the sheath in a longitudinal direction or at a position to a side of the distal end of the sheath than the central part in an opposite side of an opening face of the opening; and a guide rib including a locking protrusion for locking the cannula inside the opening with an angle, in a side view, between a line passing through an end face of the opening of the sheath and a line passing through a surface of the cannula of 0° or more with respect to a housing direction of the cannula.

Further, the present invention is preferably configured to be used for a high viscosity formulation. Further, a silicone film is preferably formed from the distal end to within a range of 60% to 85% of the length on the surface of the cannula.

Further, the sheath preferably includes, as a finger guiding region with which a finger of a user operating the sheath comes into contact, a circular recess and a slope formed between the circuit recess and the stopper, the slope having a plurality of protrusions and recesses, the circular recess and the slope being located at a side of a proximal end of the sheath than the stopper in the opposite side of the opening face of the opening.

### Advantageous Effects of Invention

The present invention makes it possible to safely house the cannula in the sheath and reliably prevent a sting accident. Further, the silicone film formed on the cannula enables the cannula to be more reliably and safely housed in the sheath.

### Brief Description of Drawings

Fig. 1 is an exploded perspective view of a safety needle assembly in an embodiment of the present invention.
Fig. 2 is a perspective view of a hub of the safety needle assembly in the embodiment of the present invention.
Fig. 3 (a) is a perspective view of a collar of the safety needle assembly in the embodiment of the present invention.
Fig. 3 (b) is a perspective view of the collar of Fig. 3 (a) viewed from a back side thereof.
Fig. 4 (a) is a perspective view of a sheath of the safety needle assembly in the embodiment of the present invention.
Fig. 4 (b) is a perspective view of the sheath of Fig. 4 (a) viewed from a bottom side thereof.
Fig. 5(a) is a perspective view illustrating a state in which a cannula is housed in the sheath of the safety needle assembly in the embodiment of the present invention.
Fig. 5 (b) is a sectional view taken along line A-A of Fig. 5(a).
Fig. 5 (c) is a sectional view taken along line B-B of Fig. 5 (b) .
Fig. 6 is a perspective view of the safety needle assembly in the embodiment of the present invention.
Fig. 7 is a perspective view of the safety needle assembly in a state ready to be used in the embodiment of the present invention.
FIG. 8 is a perspective view illustrating a state just before the cannula is housed in the sheath in the safety needle assembly in the embodiment of the present invention.

### Description of Embodiment

Hereinbelow, a safety needle assembly of the present invention will be described in detail on the basis of a preferred embodiment illustrated in the accompanying drawings.

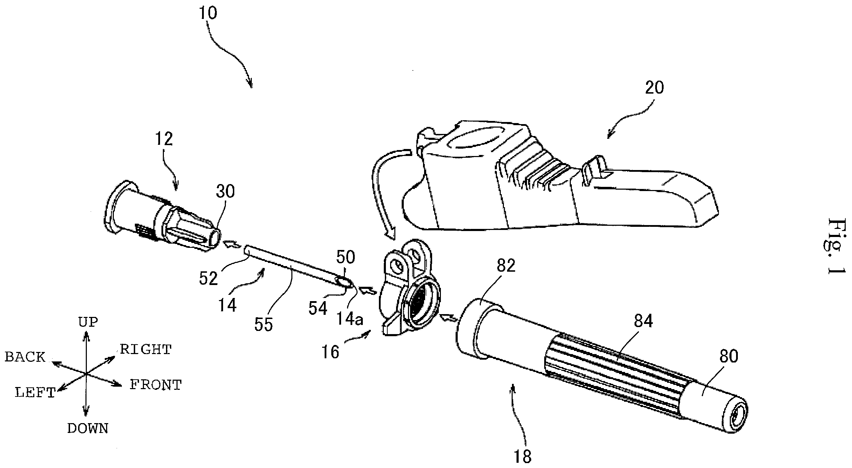

Fig. 1 is an exploded perspective view of the safety needle assembly in the embodiment of the present invention.

The safety needle assembly 10 in the present embodiment is connected to a fluid transfer device such as a syringe (hereinafter, merely referred to as "syringe") to be used.

The safety needle assembly 10 is used in subcutaneous injection of a high viscosity formulation. The high viscosity of the high viscosity formulation indicates a viscosity of 30 cP or more.

The safety needle assembly 10 is packaged, for example, in a blister packaging which is formed by extending a plastic sheet material and connected to a syringe by a user such as a health care worker when used. Alternatively, the safety needle assembly 10 may be packaged after connected to a syringe.

Note that when each component is described, a "direction" described therein is defined by the respective directions of "UP" and "DOWN", "FRONT" (distal end side) and "BACK" (proximal end side), and "LEFT" and "RIGHT" illustrated in Fig. 1. In the other drawings, the directions are similarly described to those in Fig. 1. Further, it should be noted that the "proximal end" and the "distal end" may include not only the "end", but also a peripheral portion of the "end".

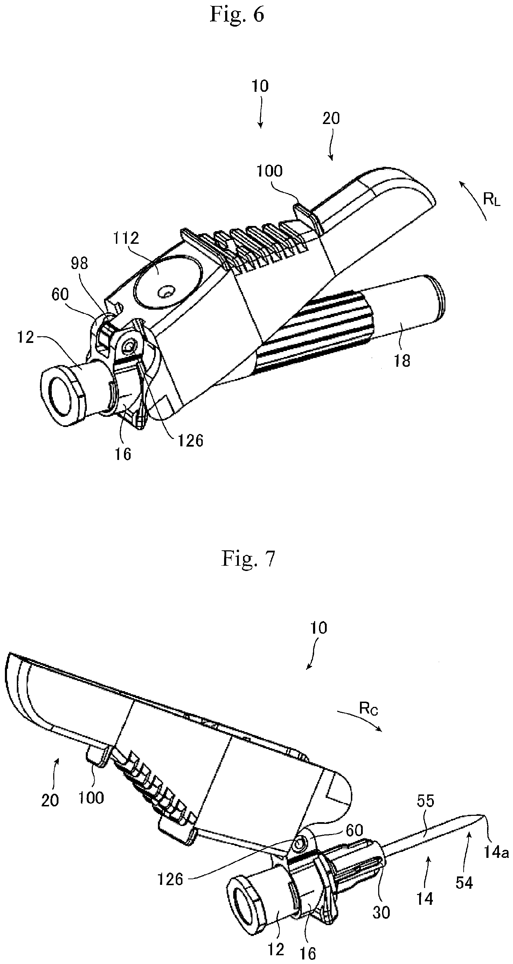

The safety needle assembly 10 illustrated in Fig. 1 includes a hub 12, a cannula 14, a collar 16, a protector 18, and a sheath 20. Note that an assembly having no protector 18 is also referred to as a "safety needle assembly 10". Fig. 6 illustrates an assembled state (described below) of the safety needle assembly 10 illustrated in Fig. 1.

The cannula 14 is connected to the hub 12 by a general method, for example, using an epoxy resin. The cannula 14 includes a proximal end 52 which is connected to a distal end 30 of the hub 12 and a distal end 54 which has a blade surface 50. The distal end 54 includes a needle point 14a.

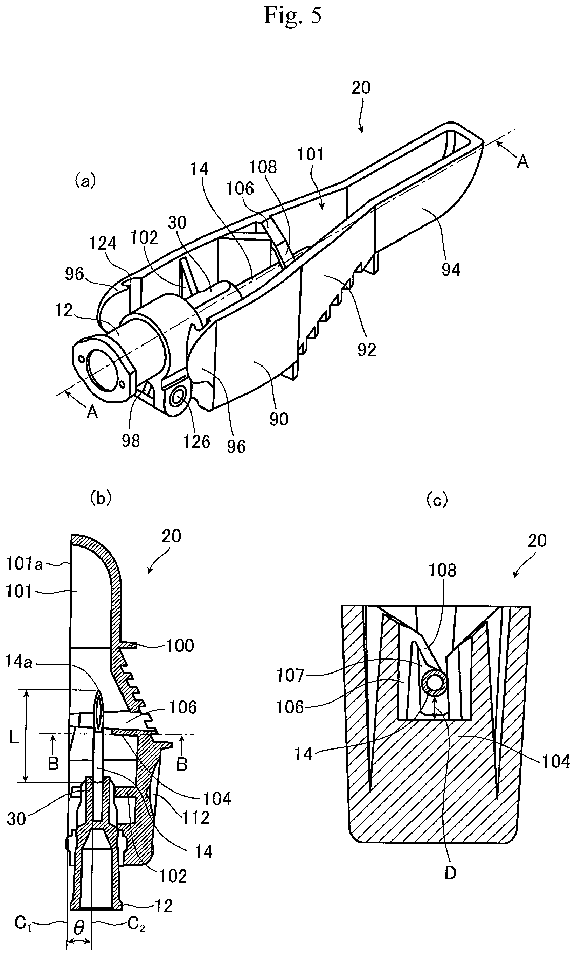

The cannula 14 has a thickness of 18 to 24 gauge and a length L between the hub 12 and the distal end 54 of 8 to 22 mm. The length L indicates the length between the distal end 54 of the cannula 14, that is, the needle point 14a and the hub 12 (refer to Fig. 5(b)).

The thickness of 18 to 24 gauge of the cannula is suitable for injection of a high viscosity formulation. When the thickness of the cannula falls out of this value range, defective conditions occur. Specifically, when the thickness of the cannula is larger than this value range, a range of damaging tissues is expanded. On the other hand, when the thickness of the cannula is smaller than this value range, it is difficult to inject a high viscosity formulation. Further, the length L between the hub 12 and the distal end 54 of 8 to 22 mm is suitable for subcutaneous injection performed at an angle of approximately 45° with respect to a skin surface. From the above viewpoints, the thickness of the cannula is more desirably 18 to 19 gauge, and the length L is more desirably 12 mm to 16 mm.

The surface of the cannula 14 is silicone-treated to form a silicone film 55 thereon. The silicone film 55 is formed within a range of 60% to 85% of the length L between the needle point 14a and the hub 12. For example, when the length L is 13 mm, the silicone film 55 is formed up to a position that is 8 mm to 11 mm from the needle point 14a. The silicone film 55 is formed by, for example, dipping.

The silicone film 55 enables a patient to feel less pain during injection. As described below, the cannula 14 is locked by a guide rib 106 (refer to Fig. 5(c)). In this locking, the silicone film 55 enables the cannula 14 to be more reliably and safely locked. When the silicone film 55 is formed within the range of 60% to 85% of the length L between the hub 12 and the distal end 54 as described above, it is possible to reduce pain during injection, and more reliably and safely lock the cannula 14 inside the sheath 20 by the guide rib 106.

It is preferred to mold the hub 12, the collar 16, the protector 18, and the sheath 20 as one-piece parts. However, the present embodiment is not limited to this configuration.

The collar 16 is attached to the hub 12 at a predetermined position. The protector 18 is guided by the hub 12 and attached to at least either the collar 16 or the hub 12 to thereby cover the cannula 14. The sheath 20 is pivotably attached to the collar 16. These components will be described in detail below.

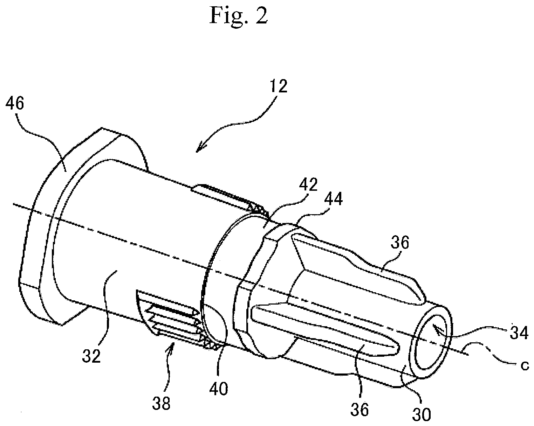

As illustrated in Fig. 2, the hub 12 includes the distal end 30 and a proximal end 32 which is connected to a syringe (not illustrated). A through hole 34 extends along the entire length of the hub 12 in a longitudinal direction (front-back direction) thereof. The through hole 34 is open at both the distal end 30 and the proximal end 32 of the hub 12. The proximal end 52 of the cannula 14 illustrated in Fig. 1 is fitted and fixed to the inside of the through hole 34 at the distal end 30 of the hub 12 by, for example, using an epoxy resin. Accordingly, a lumen extending along the entire length of the cannula 14 communicates with the through hole 34 inside the hub 12.

The distal end 30 of the hub 12 includes a plurality of stepped ribs 36 extending in the longitudinal direction. The ribs 36 facilitate coaxial attachment (in a central axis c) of the collar 16 to the hub 12.

An outer surface of the hub 12 includes a plurality of protrusions 38 extending outward in the radial direction from an outer circumferential surface of the hub 12.

A ring-shaped recessed region 40 is located at a distal side of the protrusions 38. Accordingly, step parts 42 and 44 which are adjacent to the ring-shaped recessed region 40 have larger outside diameters than the ring-shaped recessed region 40.

The proximal end 32 of the hub 12 includes a flange 46 which projects outward in the radial direction. The flange 46 is engaged with, for example, a distal end of a syringe body (not illustrated). The engagement allows the hub 12 to be connected to the syringe body (not illustrated). This connection may be performed by a known method, for example, by engaging the flange 46 with a screw (female screw) located at the distal end of the syringe body (not illustrated).

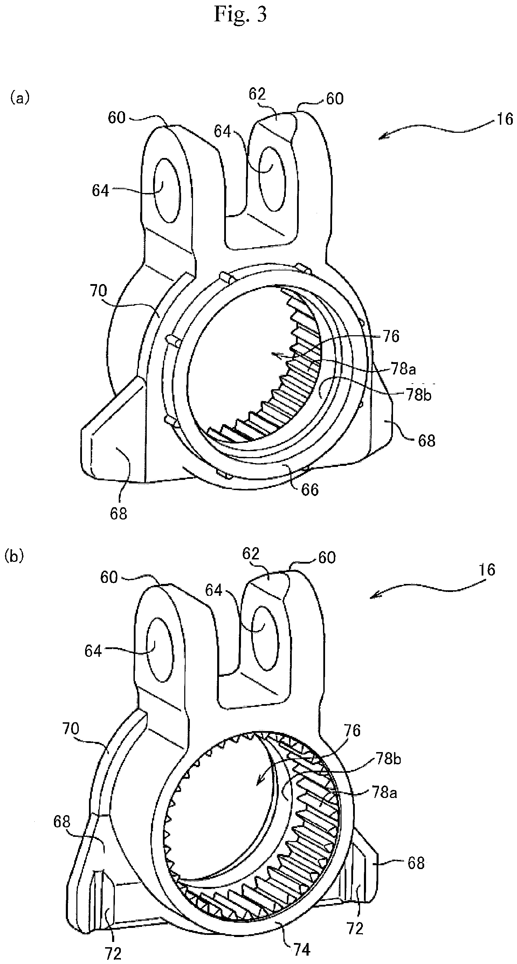

As illustrated in Figs. 3(a) and 3(b), the collar 16 has an outline in a ring shape, and has a proximal end 74 and a distal end 66. The collar 16 further includes a through hole 76 for attaching the collar 16 to the hub 12. An inside surface of the collar 16 has a plurality of protrusions 78a projecting inward. The protrusions 78a projecting inward are engaged with the protrusions 38 (refer to Fig. 2) of the hub 12. This engagement unrotatably fixes the collar 16 to the hub 12.

The collar 16 includes a ring-shaped ridge 78b projecting inward on an inner surface thereof. The ring-shaped ridge 78b extends over the whole circumference of the inner peripheral part of the through hole 76. When the collar 16 is attached to the hub 12, the ring-shaped ridge 78b is seated onto the ring-shaped recessed region 40 (refer to Fig. 2) of the hub 12. Therefore, the ring-shaped ridge 78b has an inside diameter that is smaller than the outside diameters of the two step parts 42 and 44 which are directly adjacent to the ring-shaped recessed region 40 of the hub 12 and substantially equal to the outside diameter of the ring-shaped recessed region 40 of the hub 12. The ring-shaped ridge 78b projecting inward in the radial direction is illustrated as a continuous ring-shaped ridge. However, the ring-shaped ridge 78b may be formed as a plurality of separate segments, each of which extends along a part of the inner circumference of the collar 16.

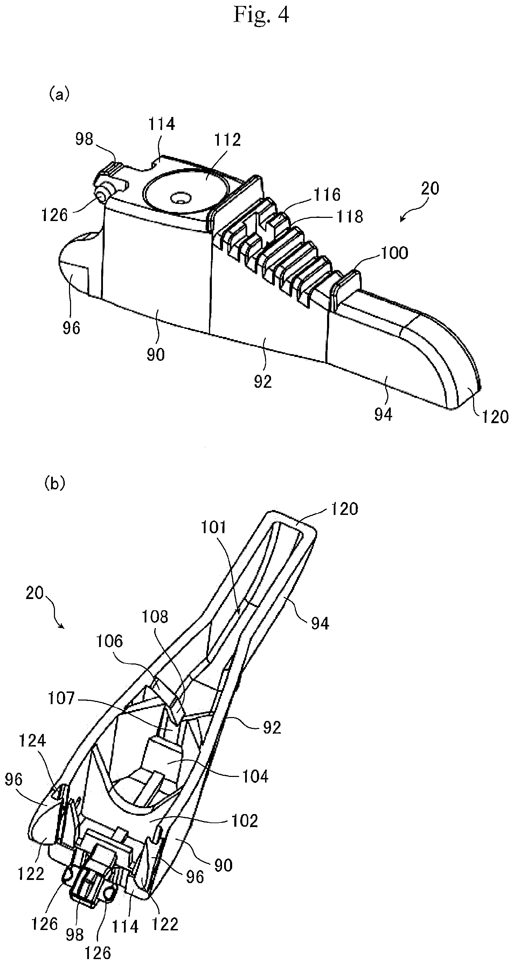

The collar 16 includes a pair of ears 60 (sheath attachment part) projecting outward in the radial direction. Each of the ears 60 includes a circular through hole 64 which houses therein a pin 126 of the sheath 20 (described below, refer to Figs. 4 (a) and 4(b)), and a chamfer 62. The chamfer 62 is formed in an upper part of the ear 60 at the inner side thereof as illustrated in Figs. 3(a) and 3(b). The pin 126 of the sheath 20 passes the chamfer 62, and is then housed in the through hole 64.

That is, the pair of ears 60 enables the sheath 20 to be rotatably attached to the collar 16. More specifically, the chamfers 62 facilitate the attachment of a pair of the pins 126 of the sheath 20 to the respective ears 60 of the collar 16. When the pair of pins 126 is attached to the pair of ears 60, the ears 60 slightly bend in directions away from each other. The thickness of each of the ears 60 may be appropriately determined taking into consideration the strength for resistance to damage and the flexibility for easier attachment of the sheath 20.

The collar 16 includes a ring-shaped ridge 70 projecting outward in the radial direction. The ring-shaped ridge 70 is a component for seating the protector 18.

The collar 16 further includes a pair of sheath fixing protrusions 68. Each of the sheath fixing protrusions 68 has a substantially triangular shape, and is disposed at the opposite side of the ear 60. The sheath fixing protrusion 68 further includes a groove 72 which is engaged with a wing wall 96 (cannula fixing part) (refer to Figs. 4 (a) and 4 (b) ) of the sheath 20.

As illustrated in Fig. 1, the protector 18 is formed as a hollowmember having an elongated cylindrical shape, including a distal end 80 and a proximal end 82. Preferably, the protector 18 is open at the proximal end 82 and closed at the distal end 80. A plurality of ribs 84 extending in the longitudinal direction are formed on the outer surface of the protector 18 in an middle part thereof so that a health care worker can easily hold the protector 18.

The protector 18 is attached at the position to cover the cannula 14 to thereby cover up the distal end of the cannula 14. At this time, the protector 18 is fixed to at least either the collar 16 or the hub 12. The protector 18 is detachable so as to expose the distal end 54 of the cannula 14. A seating face (not illustrated) for the collar 16 may be provided inside the protector 18 at the proximal end 82 thereof.

As illustrated in Fig. 4(a), the sheath 20 includes a proximal end 114 and a distal end 120, and is formed as an elongated member in the whole body. The sheath 20 includes an opening 101 which extends along at least a part of the sheath 20 in the longitudinal direction. The opening 101 is an internal space of the sheath 20. The cannula 14 is housed inside the opening 101. In the present embodiment, the opening 101 of the sheath 20 extends along the entire length in the longitudinal direction of the sheath 20. The proximal end 114 is open in the longitudinal direction of the sheath 20, while the distal end 120 is closed in the longitudinal direction of the sheath 20.

The sheath 20 includes a finger guiding part 90, a finger guiding part 92, and a distal part 94, each of which includes a pair of side walls and a back wall. A circular recess 112 is formed on the back wall of the finger guiding part 90. The circular recess 112 is used as a finger guiding region with which a finger of a user such as a health care worker operating the sheath 20 comes into contact.

The back wall of the finger guiding part 92 is formed as a stepped slope inclined downward toward the front side. On the slope, protrusions 116 and recesses 118 are repeatedly formed so as to increase the friction against a finger of a health care worker.

The back wall of the distal part 94 includes a projecting stopper 100 which prevents a finger of a health care worker pushing the sheath 20 from further moving toward the distal end 120 of the sheath 20. The stopper 100 is formed near a central part in the longitudinal direction of the sheath 20 or at a position to a side of the distal end 120 of the sheath 20 than the central part so that a larger moment is generated to smoothly pivot the sheath 20 when a health care worker pushes the sheath 20 with the finger.

The sheath 20 includes a collar connection mechanism 98 (attachment part) which has a pair of cylindrical pins 126. The pins 126 are attached to the respective through holes 64 of the ears 60 of the collar 16 so as to rotatably connect the sheath 20 to the collar 16. Each of the pins 126 is chamfered so as to facilitate the above connection.

As illustrated in Fig. 4(a), a pair of wing walls 96 is disposed at the proximal end 114 side of the finger guiding part 90. Inside each of the wing walls 96, a slope 122 is formed in the direction toward the proximal end 114. The slopes 122 enables the sheath 20 to be easily attached to the collar 16 (that is, with a relatively small force). A groove 124 (cannula fixing part) which is fitted with the corresponding sheath fixing protrusion 68 of the collar 16 is formed at the front side of the wing wall 96. When the sheath fixing protrusions 68 (refer to Fig. 3(a)) of the collar 16 are fitted with the respective grooves 124, the wing walls 96 are expanded outward just before the fitting.

As illustrated in Fig. 4(b), a recess 102, a rib 104, and the guide rib 106 are formed in the opening 101 of the sheath 20. The recess 102, the rib 104, and the guide rib 106 are arranged in this order from the finger guiding part 90 toward the distal part 94.

The recess 102 holds the distal end 30 (refer to Fig. 2) of the hub 12. The rib 104 is used for maintaining a space of the opening 101 of the sheath 20.

The guide rib 106 is used for holding the cannula 14 inside thesheath20. The guide rib 106 includes a groove 107. A locking protrusion 108 is disposed to partially close an opening of the groove 107. The locking protrusion 108 is bendable. The guide rib 106 is, for example, integrally molded with the rib 104.

Figs. 5 (a) to 5 (c) illustrate the state in which the cannula 14 is housed and locked in the sheath 20 of the safety needle assembly 10 in the embodiment of the present invention.

When the cannula 14 is inserted into the opening 101 so as to be housed in the sheath 20, the locking protrusion 108 of the guide rib 106 is bent, which allows the cannula 14 to enter the groove 107 of the guide rib 106. At this time, since the silicone film 55 is formed on the cannula 14, the resistance against the locking protrusion 108 is small. Thus, the cannula 14 can smoothly enter the groove 107. As illustrated in Fig. 5(c), after the cannula 14 has passed the locking protrusion 108, the guide rib 106 holds the cannula 14 by the locking protrusion 108 and permanently locks the cannula 14 inside the opening 101. This locking automatically occurs when the cannula 14 passes the locking protrusion 108 of the guide rib 106 and enters the groove 107. That is, this permanent locking of the cannula 14 inside the sheath 20 can be achieved merely by allowing the cannula 14 to move into the opening 101 of the sheath 20 and enter the groove 107 of the guide rib 106. The groove 107 plays a role of positioning the cannula 14 in the right-left direction thereof when the cannula 14 is introduced into the opening 101. In this case, the distal end 30 of the hub 12 is held in the recess 102.

The cannula 14 is locked inside the sheath 20 by the locking protrusion 108. In this case, when, in a side view of Fig. 5(b), an angle between a line C₁ passing through an end face 101a of the opening 101 of the sheath 20 and a line C₂ passing through the surface of the cannula 14 is denoted by θ, the cannula 14 can be locked with an angle θ of 0° or more with respect to a direction of housing the cannula into the sheath. As the angle θ increases, the cannula 14 is tilted toward the inside of the opening 101, and the tip of the cannula 14 is located at the deeper side inside the opening 101. Thus, the cannula 14 can be more reliably housed inside the opening 101. A value of the angle θ is appropriately set in accordance with the length of the cannula 14 and the shape of the inside of the sheath 20 within a range that prevents the tip of the cannula 14 from coming out of the internal space (cannula housing space) of the sheath 20 and from abutting against the inner surface of the sheath 20 or the rib 104. The range of the angle θ is desirably 0° ≤ θ ≤ 4.4°. More preferably, the range of the angle θ is desirably 0.19° ≤ θ ≤ 4.4°.

The cannula 14 having a thickness of 18 gauge to 24 gauge and a length L of 8 to 22 mm can be reliably locked by the guide rib 106 by increasing a distance D between the cannula 14 and the rib 104 illustrated in Fig. 5(c).

As described above, the silicone film 55 is formed on the cannula 14. Thus, when the cannula 14 comes into contact with the locking protrusion 108 and enters the groove 107, it is possible to reduce the resistance of the cannula 14, and more reliably and safely lock the cannula 14 inside the sheath 20.

The components described above are arranged as illustrated in Fig. 1 and assembled to obtain the safety needle assembly 10 illustrated in Fig. 6. The assembled safety needle assembly 10 illustrated in Fig. 6 may be packaged in a blister packaging or the like in the state illustrated in Fig. 6, or may be packaged after connected to a syringe (not illustrated).

Next, a method for using the safety needle assembly 10 will be described with reference to Figs. 6 to 8.

As illustrated in Fig. 6, in the safety needle assembly 10, the protector 18 is attached to the cannula 14 (not illustrated in Fig. 6). When the safety needle assembly 10 is used in subcutaneous injection of a high viscosity formulation, a health care worker detaches the protector 18 to expose the cannula 14. Thus, the health care worker first pivots the sheath 20 in a direction R_{L} so as to be apart from the protector 18.

After the sheath 20 is pivoted, the health care worker removes the protector 18 to expose the cannula 14 as illustrated in Fig. 7. In this state of the safety needle assembly 10 illustrated in Fig. 7, the health care worker can use the cannula 14, for example, for subcutaneously injecting a high viscosity formulation to a patient. In this case, the silicone film 55 enables the patient to feel less pain during the injection.

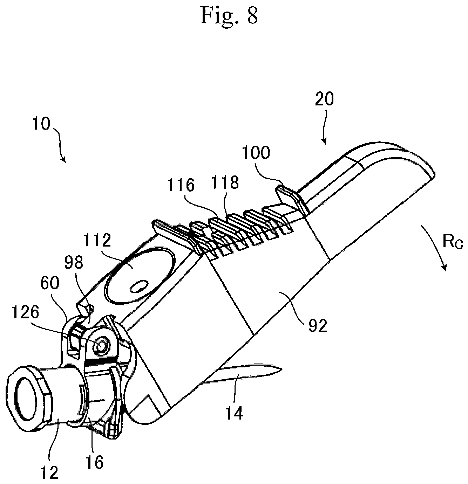

After finishing the use of the cannula 14, the health care worker first pivots the sheath 20 in a direction Rc toward the cannula 14 so that the safety needle assembly 10 becomes a state illustrated in Fig. 8 to house the cannula 14 inside the sheath 20.

Then, the health care worker pushes the back wall of the finger guiding part 92 (refer to Fig. 4 (a) ) with a finger against the sheath 20. This allows the sheath 20 to further pivot toward the cannula 14. The cannula 14 bends the locking protrusion 108 of the guide rib 106 and enters the groove 107. Accordingly, the cannula 14 is locked by the locking protrusion 108 (refer to Fig. 5(c)). At this time, the distal end 30 of the hub 12 is held in the recess 102 (refer to Fig. 5(b)). Further, the sheath fixing protrusions 68 of the collar 16 are engaged with the respective grooves 124 of the sheath 20. Accordingly, the cannula 14 is locked with respect to the sheath 20 (refer to Fig. 5(a)). That is, at this time, the cannula 14 is covered with the sheath 20, and further locked inside the sheath 20.

As described above, in the safety needle assembly 10 in the present embodiment, the cannula 14 is covered with the sheath 20 and locked by the guide rib 106. Thus, it is possible to prevent a sting accident such that a health care worker erroneously sticks the cannula 14 into the body after using the cannula 14 and thus obtain a high safety. The guide rib 106 is easily integrally molded with the rib 104. Thus, the fixing part of the cannula 14 can be achieved at a low cost.

Further, a high operability can be achieved due to the structure of the sheath 20 which has the projecting stopper 100 (refer to Fig. 4 (a) ) near the central part in the longitudinal direction of the sheath 20 or at the position to a side of the distal end of the sheath 20 than the central part and. In other words, the above positioning of the stopper 100 enables a health care worker to push the sheath 20 with a finger at a position away from the fulcrum (the position of the pins 126 of the sheath 20), resulting in generation of a larger moment which enables the sheath 20 to be pivoted with a relatively smaller force.

Further, the pair of wing walls 96 (refer to Fig. 4 (b) ) each having the groove 124 (refer to Fig. 4 (b) ) and the slope 122 (refer to Fig. 4 (b)) in the sheath 20 enables a high operability to be achieved. That is, the wing walls 96 projecting from the body of the sheath 20 are easily bent outward, and the slopes 122 facilitate introduction of the sheath fixing protrusions 68 (refer to Fig. 3(a)) of the collar 16 into the grooves 124. Further, the sheath fixing protrusions 68 can be fixed by the grooves 124. These configurations enable the sheath fixing protrusions 68 of the collar 16 to be fitted and fixed to the grooves 124 of the wing walls 96 of the sheath 20 with a relatively small force when a user such as a health care worker pushes the sheath 20 with a finger.

Further, a health care worker can push the sheath 20 not only at the slope provided with the plurality of protrusions 116 (refer to Fig. 4 (a) ) and recesses 118 (refer to Fig. 4 (a) ) having a large friction, but also at the circular recess 112 (refer to Fig. 4(a)) to which the finger easily fits. Accordingly, a high operability can be achieved.

Further, the ears 60 (refer to Fig. 3(a)) of the collar 16 (refer to Fig. 3 (a) ) have the chamfers 62 (refer to Fig. 3 (a) ) inside thereof. The sheath 20 is attached to the collar 16 through the chamfers 62, which results in a relatively small force required for the attachment.

Further, each of the sheath fixing protrusions 68 (refer to Fig. 3(a)) of the collar 16 can be achieved with a simple structure that is formed in a substantially triangle having the groove 72.

For example, if there are a plurality of cannulas 14 having different lengths, different sheaths 20 corresponding to the respective lengths may be used.

The present invention is basically configured in the above manner. Hereinabove, the safety needle assembly of the present invention has been described in detail. However, the present invention is not limited to the above embodiment. It is needless to say that various improvements and modifications may be made without departing from the scope of the invention.

### Reference Signs List

- 10: safety needle assembly
- 12: hub
- 14: cannula
- 16: collar
- 18: protector
- 20: sheath
- 104: rib
- 106: guide rib
- 107: groove

## Claims

1. A safety needle assembly comprising:
a hub including a proximal end that is configured to be connected to a syringe and a distal end;
a cannula including a proximal end that is configured to be connected to the distal end of the hub, a lumen extending in a longitudinal direction of the cannula, and a distal end, the cannula having a thickness of a range of 18 to 24 gauge and a length between the hub and the distal end of a range of 8 to 22 mm;
a collar attached to the hub and including a sheath attachment part;
a protector configured to cover the distal end of the cannula by being attached to the hub or the collar, the protector being detachable for exposing the distal end of the cannula; and
a sheath including an opening extending along at least a part of the sheath in a longitudinal direction, the opening being capable of housing the cannula,
the sheath further comprising:
a cannula fixing part configured to fix the cannula, the cannula fixing part extending along at least a part of the sheath in the longitudinal direction;
an attachment part configured to rotatably support the sheath in a direction of housing the cannula in the opening when the protector is detached to expose the distal end of the cannula, the attachment part being attached to the sheath attachment part of the collar;
a projecting stopper for restricting movement, toward a distal end of the sheath, of a finger of a user pushing the sheath for housing the cannula into the opening, the stopper being disposed near a central part of the sheath in a longitudinal direction or at a position to a side of the distal end of the sheath than the central part in an opposite side of an opening face of the opening; and
a guide rib including a locking protrusion for locking the cannula inside the opening with an angle, in a side view, between a line passing through an end face of the opening of the sheath and a line passing through a surface of the cannula of 0° or more with respect to a housing direction of the cannula.

2. The safety needle assembly according to claim 1, wherein the safety needle assembly is configured to be used for a high viscosity formulation.

3. The safety needle assembly according to claim 1 or 2, wherein a silicone film is formed from the dital end to within a range of 60% to 85% of the length on the surface of the cannula.

4. The safety needle assembly according to any one of claims 1 to 3, wherein the sheath includes, as a finger guiding region with which a finger of a user operating the sheath comes into contact, a circular recess and a slope formed between the circuit recess and the stopper, the slope having a plurality of protrusions and recesses, the circular recess and the slope being located at a side of a proximal end of the sheath than the stopper in the opposite side of the opening face of the opening.
